# EUROPEAN PATENT SPECIFICATION

(11) **EP 4 594 639 B1**
(45) Date of publication and mention of the grant of the patent: **10.12.2025**
(21) Application number: 23772842.3
(22) Date of filing: 18.09.2023
(51) Int. Cl.: F04B 23/06, F04B 43/04, F04B 49/06

(54) **SYSTEMS AND METHODS OF ACCELERATING PRESSURE AND FLOW CAPABILITY IN FLUID PUMP SYSTEMS**
SYSTEME UND VERFAHREN ZUR BESCHLEUNIGUNG DES DRUCKS UND DER FLUSSKAPAZITÄT IN FLÜSSIGKEITSPUMPENSYSTEMEN
SYSTÈMES ET PROCÉDÉS D' ACCÉLÉRATION DE CAPACITÉ DE PRESSION ET DE DÉBIT DANS DES SYSTÈMES DE POMPE À FLUIDE

(30) Priority: 27.09.2022 US 202263410411 P
(43) Date of publication of application: 06.08.2025
(73) Proprietor: Koninklijke Philips N.V., 5656 AG Eindhoven (NL)
(72) Inventor: KOSTAKIS, Dimitri George, 5656 AG Eindhoven (NL); APPLETON, Bruce Geoffrey, 5656 AG Eindhoven (NL)
(74) Representative: Philips Intellectual Property & Standards
(86) International application number: PCT/EP2023/075556
(87) International publication number: WO 2024/068318

(56) References cited:
- CN-A- 113 738 623
- GB-A- 2 585 497
- US-A1- 2015 059 749

## Description

### Field of the Disclosure

The present disclosure relates generally to fluid flow systems and methods, and more specifically to fluid flow systems and methods utilizing a combination of specialized pumps to achieve a desired performance.

### Background

Fluid pumps are used alone or in combination with other pumps in a fluid flow system to achieve a desired pressure curve within a required amount of time. However, depending on the intended application of the fluid flow system, certain limitations may be placed on the types of fluid pumps and/or materials used to construct the fluid flow system. For example, when used in medical devices, these limitations can include safety- and/or performance-related requirements, such as minimum- or maximum-rated pressures, flowrates, voltage and current specifications, and the like. These limitations can also include an operability requirement, such as requiring that the fluid flow system operate in environments subjected to high magnetic fields (*e.g.,* in a magnetic resonance imaging suite, etc.). While conventional pumps, such as conventional motor-driven diaphragm (also known as rolling pumps) may meet many of the performance-related requirements, these conventional pumps contain materials and/or mechanisms that are negatively affected by high magnetic fields, making them unacceptable for use in certain medical devices. US2015059749A1 relates to a pump unit for transporting a fluid by means of a micro pump and a respiratory assistance device employing the same. GB2585497A relates to a fluid control device that uses a piezoelectric pump to move fluids to a predetermined direction.

### Summary of the Disclosure

According to an embodiment of the present disclosure, a fluid flow system for use in a medical device is provided. The fluid flow system comprises: a flow pathway comprising one or more fluid inputs and a fluid output, the fluid output being connectable with an inflatable device configured to receive the fluid output; a first pump configured to receive a first fluid input and output a first intermediate fluid flow; and a second pump operatively connected in series to the first pump, the second pump being configured to receive at least a portion of the first intermediate fluid flow and output a second intermediate fluid flow. In embodiments, the fluid output comprises the second intermediate fluid flow and has an output flowrate such that the inflatable device reaches a target pressure within a target duration.

In an aspect, the first pump and the second pump are rated for use in magnetic fields exceeding 1000 gauss.

In an aspect, at least one of the first pump and the second pump is a piezoelectric pump.

According to the invention, the flow pathway includes a second fluid input between the first pump and the second pump, and wherein the second pump is configured to receive the first intermediate fluid flow and the second fluid input.

According to the invention, the system further comprises a check valve system along the flow pathway between the first pump and the second pump, the check valve system being configured to regulate the second fluid input and prevent back flow along the flow pathway.

In an aspect, the first pump has a first peak pressure that is greater than a second peak pressure of the second pump, the first pump has a first maximum flowrate that is less than a second maximum flowrate of the second pump, and the target pressure is greater than each of the first peak pressure and the second peak pressure.

In an aspect, the flow pathway includes a flow bypass between the first pump and the second pump, the flow bypass configured to divert at least a portion of the first intermediate fluid flow before the second pump and feed the diverted portion of the first intermediate fluid flow back into a downstream portion of the flow pathway located after the second pump such that the fluid output comprises the second intermediate fluid flow and the diverted portion of the first intermediate fluid flow.

In an aspect, the system further comprises a check valve system along the flow bypass of the flow pathway, the check valve system being configured to regulate the diverted portion of the first intermediate fluid flow and prevent back flow along the flow pathway.

In an aspect, the first pump has a first peak pressure that is less than a second peak pressure of the second pump, the first pump has a first maximum flowrate that is greater than a second maximum flowrate of the second pump, and the target pressure is greater than each of the first peak pressure and the second peak pressure.

In an aspect, the target pressure is at least about 250 mmHg and the target duration is at most about 30 seconds.

According to another embodiment of the present disclosure, a medical device according to claim 9 is provided.

In an aspect, the medical device is at least one of: an MRI-compatible medical monitor; an anesthesia cart; a bedside monitor; a non-invasive blood pressure monitor; and a non-invasive gas monitor.

According to yet another embodiment of the present disclosure, a method of regulating delivery of a fluid flow in a medical device coupled to a patient is provided. The method comprises: flowing at least a first fluid input through a first pump of a fluid flow system along a fluid pathway to generate a first intermediate fluid flow; flowing at least a portion of the first intermediate fluid flow through a second pump of the fluid flow system along the fluid pathway to generate a second intermediate fluid flow; and outputting from the fluid flow system a fluid output to an inflatable device connected to the fluid flow system, the fluid output comprising the second intermediate fluid flow. In embodiments, the fluid output has an output flowrate such that the inflatable device reaches a target pressure within a target duration.

According to the invention, the method further comprises: flowing a second fluid input through a check valve system along the fluid pathway between the first pump and the second pump such that the second pump received the first intermediate fluid flow and the second fluid input.

In an aspect, the method further comprises: diverting at least a portion of the first intermediate fluid flow through a flow bypass along the flow pathway; and introducing the diverted portion of the first intermediate fluid flow back into a downstream portion of the flow pathway located after the second pump, wherein the fluid output comprises the second intermediate fluid flow and the diverted portion of the first intermediate fluid flow.

These and other aspects of the various embodiments will be apparent from and elucidated with reference to the embodiments described hereinafter.

### Brief Description of the Drawings

In the drawings, like reference characters generally refer to the same parts throughout the different views. Also, the drawings are not necessarily to scale, emphasis instead generally being placed upon illustrating the principles of the various embodiments.
FIG. 1 is a block diagram of a fluid flow system illustrated according to aspects of the present disclosure.
FIG. 2 is a schematic diagram of a fluid flow system having two pumps illustrated according to aspects of the present disclosure.
FIG. 3 is a schematic diagram of another fluid flow system having two pumps illustrated according to aspects of the present disclosure.
FIG. 4 is a graph of the fluid output pressure versus time illustrated according to aspects of the present disclosure.
FIG. 5 is a diagram of a medical device comprising a fluid flow system illustrated according to aspects of the present disclosure.
FIG. 6 is a flowchart of a method for regulating delivery of a fluid flow in a medical device coupled to a patient illustrated according to aspects of the present disclosure.

### Detailed Description of Embodiments

The present disclosure is directed to fluid flow systems and methods adapted for use in specialized environments. More specifically, the present disclosure is directed to fluid flow systems and methods that involve multiple fluid pumps configured to operate in environments subjected to high magnetic fields. While conventional motor-driven diaphragm pumps, also known as rolling pumps, may meet many of the safety- and/or performance-related requirements, systems and methods incorporating such pumps are not suitable for use in high magnetic field environments. In contrast, individual fluid pumps that have been adapted for use in high magnetic field environments may not meet one or more of the safety- and/or performance-related requirements. As described herein, the fluid flow systems and methods achieve one or more desired performance-related specifications while still being suitable for use in high magnetic field environments.

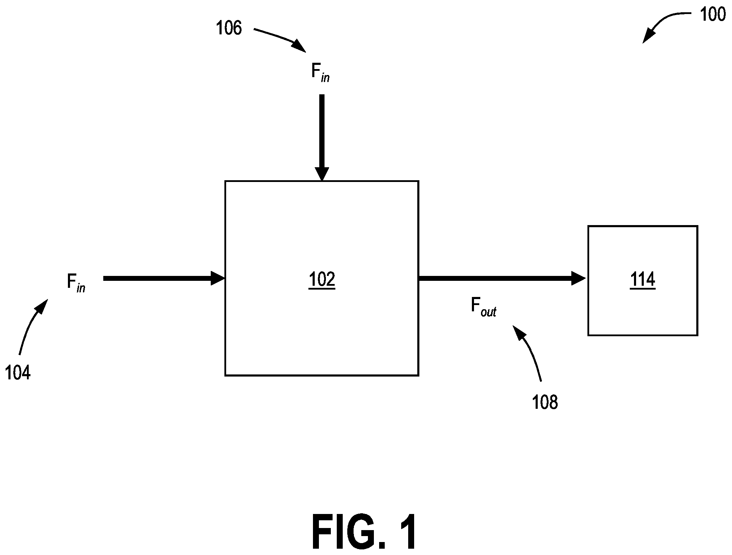

Turning to FIG. 1, a block diagram of a fluid flow system 100 is illustrated according to aspects of the present disclosure. In the example of FIG. 1, the fluid flow system 100 comprises an arrangement 102 of components and a fluid flow pathway comprising one or more fluid inputs (Fᵢₙ) 104, 106 and a fluid output (Fₒᵤₜ) 108. As shown, the fluid flow system 100 is operatively connected to an inflatable device 114 configured to receive the fluid output (Fₒᵤₜ) 108 from the fluid flow system 100. In embodiments, the arrangement 102 of components can include two or more pumps, including two or more pumps operatively connected in series. As such, the arrangement 102 of components is configured to receive one or more fluid flow inputs (Fᵢₙ) 104, 106 and output a fluid output (Fₒᵤₜ) 108.

The one or more fluid inputs (Fᵢₙ) includes at least a first fluid input (Fᵢₙ) 104. In embodiments, the one or more fluid inputs (Fᵢₙ) includes at least a second fluid input (Fᵢₙ) 106. Each of the one or more fluid inputs (Fᵢₙ) 104, 106 may be taken from the same source or may be taken from different sources. In certain embodiments, one or more of the fluid inputs (Fᵢₙ) 104, 106 is ambient air. In other embodiments, one or more of the fluid inputs (Fᵢₙ) 104, 106 comprises a gas mixture other than ambient air. In some embodiments, the one or more of the fluid inputs (Fᵢₙ) 104, 106 includes a pharmaceutical composition or a combination of pharmaceutical compositions.

The fluid flow system 100 includes at least one fluid output (Fₒᵤₜ) 108, which is a fluid flow comprising at least a portion of the one or more fluid inputs (Fᵢₙ) 104, 106. In certain embodiments, fluid output (Fₒᵤₜ) 108 comprises ambient air. In other embodiments, the fluid output (Fₒᵤₜ) 108 includes a pharmaceutical composition or a combination of pharmaceutical compositions. In embodiments, the fluid output (Fₒᵤₜ) 108 may comprise a gas mixture other than ambient air.

In embodiments, the fluid output (Fₒᵤₜ) 108 has an output flowrate that is greater than the input flowrate(s) of the one or more of the fluid inputs (Fᵢₙ) 104, 106. As discussed in detail below, the fluid flow system 100 can be configured such that inflatable device 114 connected to the fluid output (Fₒᵤₜ) 108 reaches a target pressure within a target duration.

In the example of FIG. 1, the arrangement 102 of components can include one or more components that are rated for use in high magnetic fields or are otherwise operable in high magnetic field environments. For example, the arrangement 102 of components may include two or more pumps that are configured to operate in high magnetic field environments. According to the present disclosure, a high magnetic field environment can be an environment where the magnetic flux density is greater than about 1000 gauss (or about 0.1 tesla), including greater or equal to than about 5,000 gauss (i.e., about 0.5 tesla), greater than or equal to about 10,000 gauss (i.e., about 1 tesla), and greater than or equal to about 15,000 gauss (i.e., about 1.5 tesla).

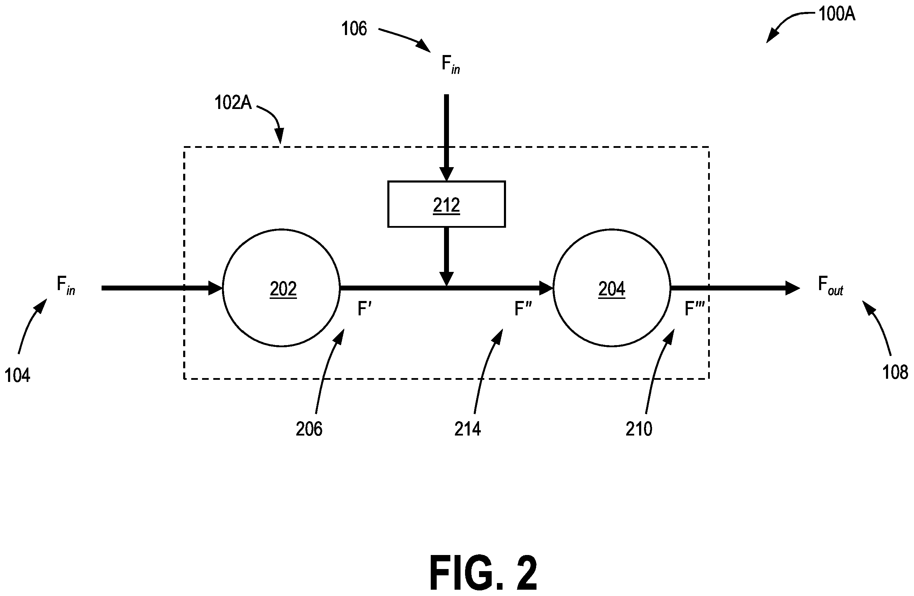

Turning to FIG. 2, a schematic block diagram of a fluid flow system 100A for use in a medical device is illustrated according to a first embodiment of the present disclosure. The system 100A includes an arrangement 102A of components and a flow pathway comprising one or more fluid inputs (Fᵢₙ) 104, 106 and a fluid output (Fₒᵤₜ) 108 that flows through the arrangement 102A of components. In the example of FIG. 2, the fluid flow system 100A comprises a first pump 202 operatively connected in series to a second pump 204. The first pump 202 is configured to receive a first fluid input (Fᵢₙ) 104 along the flow pathway and output a first intermediate fluid flow 206. The second pump 204 is configured to receive at least a portion of the first intermediate fluid flow 206 and output a second intermediate fluid flow 210.

Although the system 100A shown in FIG. 2 includes only two pumps 202, 204, it is contemplated that the system 100A can comprise two or more pumps 202, 204, including a plurality of pumps 202, 204. In embodiments, each of these pumps 202, 204 can be connected in series with one another. As discussed above, each of the pumps 202, 204 may be rated for use in high magnetic field environments. In certain embodiments, one or more of the pumps 202, 204 is a piezoelectric pump. Additionally, the one or more pumps 202, 204 may be designed to run with between 0 and 1 watt of power.

In embodiments, the first pump 202 is a high-pressure pump 202 while the second pump 204 is a high-flowrate pump 204. That is, the first pump 202 can have a first peak pressure (i.e., peak operating pressure) that is greater than a second peak pressure (i.e., peak operating pressure) of the second pump 204, while the first pump 202 has a first maximum flowrate that is less than a second maximum flowrate of the second pump 204.

As used herein, the terms "peak pressure" and "peak operating pressure" refer to the pressure at which the pump can no longer produce additional fluid flow. For example, according to certain embodiments, the high-pressure pump 202 may have a first peak pressure of from about 150 mmHg to about 230 mmHg, including about 150 mmHg, about 160 mmHg, about 170 mmHg, about 180 mmHg, about 190 mmHg, about 200 mmHg, about 210 mmHg, about 220 mmHg, and about 230 mmHg. In further embodiments, the high-flowrate pump 204 may have a second peak pressure of from about 80 mmHg to about 160 mmHg, including about 80 mmHg, about 90 mmHg, about 100 mmHg, about 110 mmHg, about 120 mmHg, about 130 mmHg, about 140 mmHg, about 150 mmHg, and about 160 mmHg.

Additionally, as used herein, the term "maximum flowrate" refers to the maximum flowrate at which a pump is able to output a fluid flow. For example, according to certain embodiments, the high-pressure pump 202 may have a first maximum flowrate of from about 0.7 liters/minute to about 1.0 liter/minute, including about 0.7 liters/minute, about 0.75 liters/minute, about 0.8 liters/minute, about 0.85 liters/minute, about 0.9 L/minute, about 0.95 liters/minute, and about 1.0 liters/minute. In further embodiments, the high-flowrate pump 204 may have a second maximum flowrate of from about 1.3 liters/minute to about 2.0 liters/minute, including about 1.3 liters/minute, about 1.4 liters/minute, about 1.5 liters/minute, about 1.6 liters/minute, about 1.65 liters/minute, about 1.7 liters/minute, about 1.8 liters/minute, about 1.9 liters/minute, and about 2.0 liters/minute.

In embodiments, the second pump 204 of the system 100A may achieve an increased fluid flow by including at least a second fluid input 106 along the fluid flow pathway of the system 100A between the first pump 202 and the second pump 202. As such, the second pump 204 can be configured to receive at least a portion of the first intermediate fluid flow 206 and the second fluid input (Fᵢₙ) 106. The fluid inputs (Fᵢₙ) 104, 106 may be taken from the same source or may be taken from different sources, as discussed above. As such, the flowrates of the first and second fluid input (Fᵢₙ) 104, 106 may be the same or may be different.

In embodiments, the second fluid input (Fᵢₙ) 106 is introduced into the fluid flow pathway of the system 100A via a check valve system 212. The check valve system 212 is configured to regulate the second fluid input (Fᵢₙ) 106 into the fluid flow pathway and to prevent back flow along the flow pathway. In embodiments, the check valve system 212 comprises one or more valves, such as one or more one-way valves.

Using the check valve system 212, the second fluid input (Fᵢₙ) 106 may be introduced to the portion of the fluid flow pathway containing the first intermediate fluid flow 206 such that the first intermediate fluid flow 206 mixes with the second fluid input (Fᵢₙ) 106 to form a blended fluid flow 214. The blended fluid flow 214 may then be received by the second pump 204. Put another way, the second intermediate fluid flow 210 includes a mixture of the first intermediate fluid flow 206 and the second fluid input (Fᵢₙ) 106.

In some embodiments, the fluid output (Fₒᵤₜ) 108 comprises the second intermediate fluid flow 210. In specific embodiments, the second intermediate fluid flow 210 is the fluid output (Fₒᵤₜ) 108, such that the output flowrate is the flowrate of the second intermediate fluid flow.

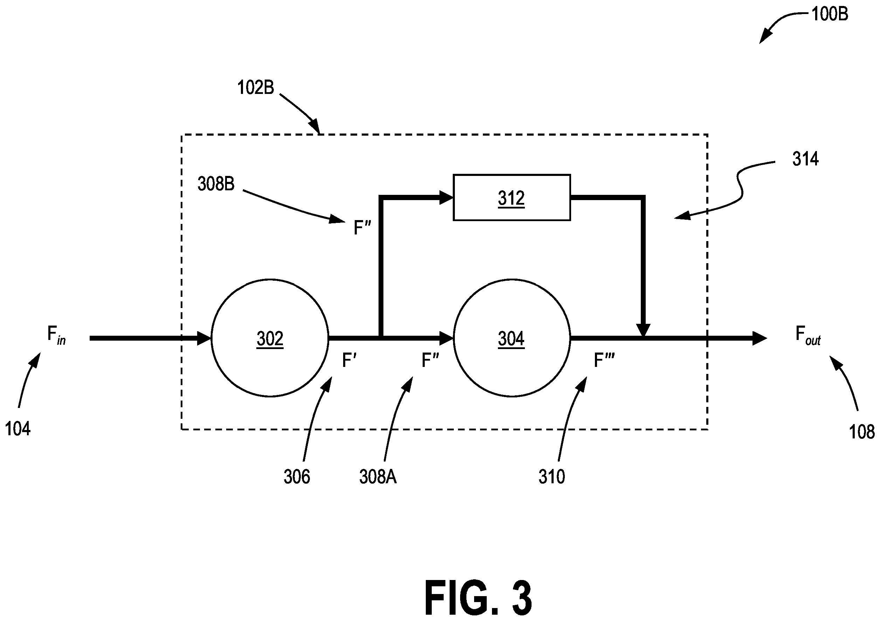

Turning to FIG. 3, a schematic block diagram of a fluid flow system 100B for use in a medical device is illustrated according to a second embodiment of the present disclosure. The system 100B includes an arrangement 102B of components and a flow pathway comprising one or more fluid inputs (Fᵢₙ) 104 and a fluid output (Fₒᵤₜ) 108 that flows through the arrangement 102B of components. In the example of FIG. 3, the fluid flow system 100B comprises a first pump 302 operatively connected in series to a second pump 304. The first pump 302 is configured to receive a first fluid input (Fᵢₙ) 104 along the flow pathway and output a first intermediate fluid flow 306. The second pump 304 is configured to receive at least a portion 308A of the first intermediate fluid flow 306 and output a second intermediate fluid flow 310.

Although the system 100B shown in FIG. 3 includes only two pumps 302, 304, it is contemplated that the system 100B can comprise two or more pumps 302, 304, including a plurality of pumps 302, 304. In embodiments, each of these pumps 302, 304 can be connected in series with one another. As discussed above, each of the pumps 302, 304 may be rated for use in high magnetic field environments, such as environments subject to magnetic fields with a magnetic flux density of at least 1000 gauss (or about 0.1 tesla). In certain embodiments, one or more of the pumps 302, 304 is a piezoelectric pump.

In embodiments, the first pump 302 is a high-flowrate pump 302 and the second pump 304 is a high-pressure pump 304. That is, the first pump 302 may have a first peak pressure (i.e., peak operating pressure) that is less than a second peak pressure (i.e., peak operating pressure) of the second pump 304, while the first pump 302 has a first maximum flowrate that is greater than a second maximum flowrate of the second pump 304.

According to certain embodiments, the high-flowrate pump 302 may have a first maximum flowrate of from about 1.3 liters/minute to about 2.0 liters/minute, including about 1.3 liters/minute, about 1.4 liters/minute, about 1.5 liters/minute, about 1.6 liters/minute, about 1.65 liters/minute, about 1.7 liters/minute, about 1.8 liters/minute, about 1.9 liters/minute, and about 2.0 liters/minute. In further embodiment, the high-pressure pump 304 may have a second maximum flowrate of from about 0.7 liters/minute to about 1 liter/minute, including about 0.7 liters/minute, about 0.75 liters/minute, about 0.8 liters/minute, about 0.85 liters/minute, about 0.9 L/minute, about 0.95 liters/minute, and about 1.0 liters/minute. In further embodiments,

According to further embodiments, the high-flowrate pump 302 may have a first peak pressure of from about 80 mmHg to about 160 mmHg, including about 80 mmHg, about 90 mmHg, about 100 mmHg, about 110 mmHg, about 120 mmHg, about 130 mmHg, about 140 mmHg, about 150 mmHg, and about 160 mmHg. In still further embodiments, the high-pressure pump 304 may have a second peak pressure of from about 150 mmHg to about 230 mmHg, including about 150 mmHg, about 160 mmHg, about 170 mmHg, about 180 mmHg, about 190 mmHg, about 200 mmHg, about 210 mmHg, about 220 mmHg, and about 230 mmHg.

In embodiments, the second pump 304 is configured to receive at least a portion 308A of the first intermediate fluid flow 306 at a first intermediate pressure and output a second intermediate fluid flow 310. In particular embodiments, the system 100A may include a flow bypass 314 that is configured to divert at least a portion 308B of the first intermediate fluid flow 306 before reaching the second pump 304. In embodiments, the flow bypass 314 is configured to feed the diverted portion 308B of the first intermediate fluid flow 306 back into a downstream portion of the flow pathway located after the second pump.

In further embodiments, the diverted portion 308B of the first intermediate fluid flow 306 is fed-forward to a portion of the flow pathway bypassing the second pump 304 such that the second intermediate fluid flow 310 and the diverted portion 308B of the first intermediate fluid flow 306 form a blended fluid flow. In embodiments, this blended fluid flow is the output fluid (Fₒᵤₜ) 108, as shown in the example of FIG. 3. Put another way, the fluid output (Fₒᵤₜ) 108 includes a mixture of the second intermediate fluid flow 310 and a diverted portion 308B of the first intermediate fluid flow 306.

In embodiments, the flow bypass 314 includes a check valve system 312 configured to regulate the diversion of a portion 308B of the first intermediate fluid flow 306 and to prevent back flow along the flow pathway. In embodiments, the check valve system 312 comprises one or more valves, such as one or more one-way valves.

According to the present disclosure, the systems 100, 100A, 100B described herein achieve one or more desired performance-related specifications while still being suitable for use in high magnetic field environments. In particular, the systems 100, 100A, 100B are configured to deliver a fluid output (Fₒᵤₜ) 108 to an inflatable device 114 such that the inflatable device 114 reaches a target pressure within a target duration. According to the present disclosure, the target pressure may be greater than each of the first peak pressure of the first pump and the second peak pressure of the second pump. Additionally, it should be appreciated that the term "target duration" as used herein refers to the time it takes the fluid flow system 100 to begin receiving the one or more fluid inputs (Fᵢₙ) 104, 106 and output the fluid output (Fₒᵤₜ) 108 at the target pressure.

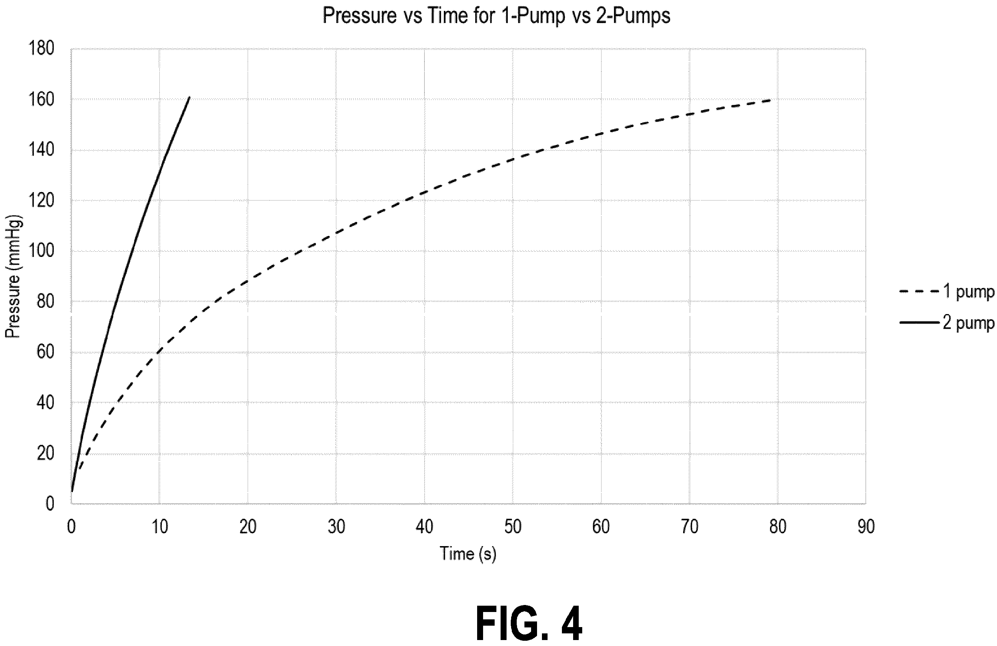

For example, with reference to FIG. 4, a graph is illustrated showing the difference between a single pump system and a multi-pump system (e.g., systems 100, 100A, 100B) according to the present disclosure. As shown, the single pump system takes over one minute to reach a pressure of 160 mmHg. In contrast, the multi-pump system reaches a pressure of 160 mmHg in less than 20 seconds.

In embodiments, the target pressure for the system 100 may be from about 80 mmHg to about 300 mmHg, including at least about 100 mmHg, at least about 110 mmHg, at least about 120 mmHg, at least about 130 mmHg, at least about 140 mmHg, at least about 150 mmHg, at least about 160 mmHg, at least about 170 mmHg, at least about 180 mmHg, at least about 190 mmHg, at least about 200 mmHg, at least about 210 mmHg, at least about 220 mmHg, at least about 230 mmHg, at least about 240 mmHg, at least about 250 mmHg, at least about 260 mmHg, at least about 270 mmHg, at least about 280 mmHg, at least about 290 mmHg, and at least about 300 mmHg.

In further embodiments, the target duration for the system 100 to achieve the target pressure may be less than about 30 seconds, including less than about 25 seconds, less than about 20 seconds, less than about 15 seconds, and less than about 10 seconds.

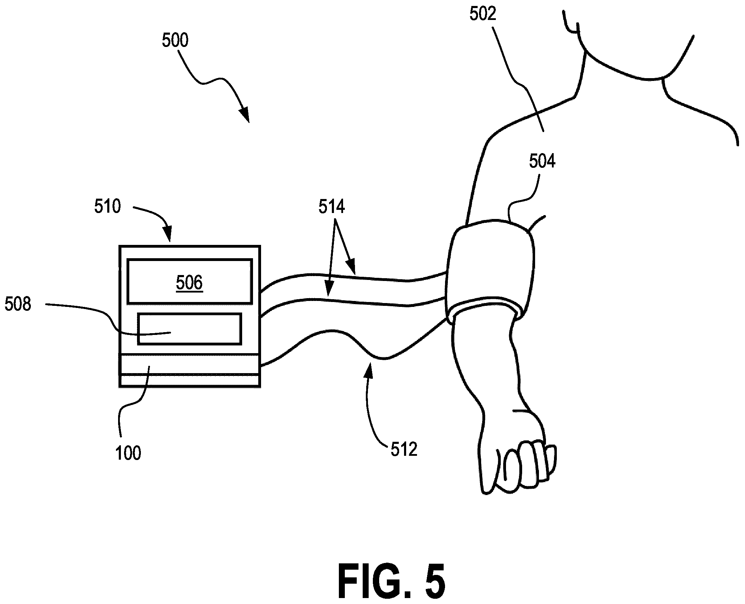

Also provided herein are medical devices comprising a fluid flow system as described above and configured to regulate delivery of a fluid flow to a patient. For example, with reference to FIG. 5, a medical device 500 comprising a fluid flow system 100 used to regulate the delivery of a fluid flow to a patient 502 is illustrated according to aspects of the present disclosure. In particular examples, the medical device 500 is a non-invasive blood pressure cuff configured to measure the patient's 502 blood pressure.

In the example of FIG. 5, the device 500 further comprises an inflatable cuff 504 (i.e., an inflatable device 114), one or more sensors (not shown) configured to sense a pressure signal indicative of a pressure within the cuff and/or pulsating blood flow in an artery of the patient 502, optionally a display 506, and a controller 508 configured to operate the fluid flow system 100 and/or the display 506, and to determine one or more physiological measurements (e.g., blood pressure, etc.) based on the signals received from the one or more sensors.

In embodiments, the fluid flow system 100, the display 506, the controller 508, and/or one or more sensors (not shown) may be contained within an external housing 510. The fluid flow system 100 may be operatively connected to the inflatable cuff 504 via a hose 512. In embodiments, the medical device 500 further comprises one or more additional data cables 514 connecting the components of within the external housing 510 to a portion of the device 500 coupled to the patient 502 (*e.g.,* the inflatable cuff 504, etc.).

Although FIG. 5 illustrates one embodiment of a medical device 500 comprising a fluid flow system 100 in accordance with the present disclosure, it is contemplated that other medical devices 500 may include the fluid flow systems 100, 100A, 100B as described herein. In particular, according to various embodiments, the medical device 500 can be at least one of the following: an MRI-compatible medical monitor; an anesthesia cart; a bedside monitor; a non-invasive blood pressure monitor; and a non-invasive gas monitor.

In embodiments, the medical device 500 including the fluid flow system 100 is particularly adapted for use in environments subjected to high magnetic fields, and may be configured to regulate delivery of a fluid flow such that the inflatable cuff 504 achieves and/or maintains a target pressure within a target duration. According to the present disclosure, the target pressure may be greater than each of the first peak pressure of the first pump and the second peak pressure of the second pump.

In specific embodiments, the target pressure may be from about 80 mmHg to about 300 mmHg, including at least about 100 mmHg, at least about 110 mmHg, at least about 120 mmHg, at least about 130 mmHg, at least about 140 mmHg, at least about 150 mmHg, at least about 160 mmHg, at least about 170 mmHg, at least about 180 mmHg, at least about 190 mmHg, at least about 200 mmHg, at least about 210 mmHg, at least about 220 mmHg, at least about 230 mmHg, at least about 240 mmHg, at least about 250 mmHg, at least about 260 mmHg, at least about 270 mmHg, at least about 280 mmHg, at least about 290 mmHg, and at least about 300 mmHg. In embodiments, the target duration may be less than about 30 seconds, including less than about 25 seconds, less than about 20 seconds, less than about 15 seconds, and less than about 10 seconds.

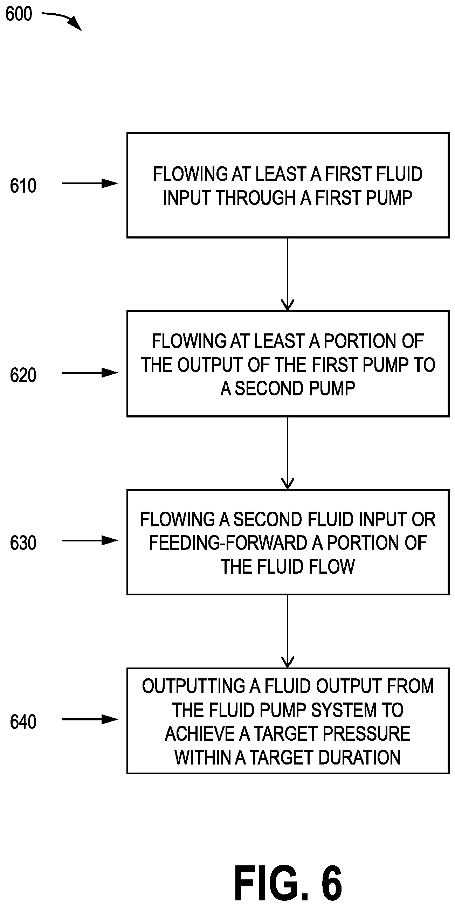

Turning to FIG. 6, also provided herein are methods 600 of regulating delivery of a fluid flow in a medical device to a subject (e.g., devices 500 and patient / subject 502).

In a step 610, the method 600 includes flowing at least a first fluid input through a first pump of a fluid flow system along a fluid pathway of the fluid flow system to generate a first intermediate fluid flow.

In a step 620, the method 600 includes flowing at least a portion of the first intermediate fluid flow through a second pump of the fluid flow system along the fluid pathway of the fluid flow system to generate a second intermediate fluid flow.

In some embodiments, the method 600 includes a step 630 of flowing a second fluid input through a check valve system along the fluid pathway of the fluid flow system between the first pump and the second pump. The second fluid input may be received by the second pump such that the second intermediate fluid flow and/or the fluid output comprises a mixture or blend of both the first intermediate fluid flow and the second fluid input.

In other embodiments, the step 630 includes: (i) diverting at least a portion of the first intermediate fluid flow through a flow bypass along the flow pathway of the fluid flow system; and (ii) introducing the diverted portion of the first intermediate fluid flow back into a downstream portion of the flow pathway located after the second pump. In such embodiments, the second intermediate fluid flow only includes a non-diverted portion of the first intermediate fluid flow, but the fluid output includes a mixture or blend of the second intermediate fluid flow and the diverted portion of the first intermediate fluid flow.

In a step 640, the method 600 includes outputting a fluid output from the fluid flow system that comprises at least the second intermediate fluid flow. In embodiments, the fluid output has an output flowrate such that the inflatable device coupled to the patient achieves a target pressure within a target duration. According to the present disclosure, the target pressure may be greater than each of the first peak pressure of the first pump and the second peak pressure of the second pump.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

It should also be understood that, unless clearly indicated to the contrary, in any methods claimed herein that include more than one step or act, the order of the steps or acts of the method is not necessarily limited to the order in which the steps or acts of the method are recited.

Other implementations are within the scope of the following claims and other claims to which the applicant can be entitled.

While several inventive embodiments have been described and illustrated herein, those of ordinary skill in the art will readily envision a variety of other means and/or structures for performing the function and/or obtaining the results and/or one or more of the advantages described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein.

## Claims

1. A fluid flow system (100, 100A, 100B) for use in a medical device (500), the fluid flow system comprising:
a flow pathway comprising one or more fluid inputs (104, 106) and a fluid output (108), the fluid output being connectable with an inflatable device (114) configured to receive the fluid output;
a first pump (202, 302) configured to receive a first fluid input (104) and output a first intermediate fluid flow (206); and
a second pump (204, 304) operatively connected in series to the first pump, the second pump being configured to receive at least a portion of the first intermediate fluid flow and output a second intermediate fluid flow (201, 310);
wherein the flow pathway includes a second fluid input (106) between the first pump and the second pump, wherein the second pump is configured to receive the first intermediate fluid flow and the second fluid input; and wherein the fluid output comprises the second intermediate fluid flow and has an output flowrate such that the inflatable device reaches a target pressure within a target duration,
**characterized by** further comprising a check valve system (212) along the flow pathway between the first pump and the second pump, the check valve system being configured to regulate the second fluid input and prevent back flow along the flow pathway.

2. The fluid flow system of claim 1, wherein the first pump and the second pump are rated for use in magnetic fields exceeding 1000 gauss.

3. The fluid flow system of claim 1, wherein at least one of the first pump and the second pump is a piezoelectric pump.

4. The fluid flow system of claim 1, wherein the first pump has a first peak pressure that is greater than a second peak pressure of the second pump, the first pump has a first maximum flowrate that is less than a second maximum flowrate of the second pump, and the target pressure is greater than each of the first peak pressure and the second peak pressure.

5. The fluid flow system of claim 1, wherein the flow pathway includes a flow bypass (314) between the first pump and the second pump, the flow bypass configured to divert at least a portion of the first intermediate fluid flow before the second pump and feed the diverted portion of the first intermediate fluid flow back into a downstream portion of the flow pathway located after the second pump such that the fluid output comprises the second intermediate fluid flow and the diverted portion of the first intermediate fluid flow.

6. The fluid flow system of claim 1, further comprising a check valve system along the flow bypass of the flow pathway (314), the check valve system being configured to regulate the diverted portion of the first intermediate fluid flow and prevent back flow along the flow pathway.

7. The fluid flow system of claim 6, wherein the first pump has a first peak pressure that is less than a second peak pressure of the second pump, the first pump has a first maximum flowrate that is greater than a second maximum flowrate of the second pump, and the target pressure is greater than each of the first peak pressure and the second peak pressure.

8. The fluid flow system of claim 1, wherein the target pressure is at least about 250 mmHg and the target duration is at most about 30 seconds.

9. A medical device (500) comprising a fluid flow system (100, 100A, 100B) according to claims 1-8, configured to produce a fluid output and an inflatable device (114) configured to receive the fluid output, the fluid flow system being operatively connected to the inflatable device.

10. The medical device of claim 9, wherein the medical device is at least one of: an MRI-compatible medical monitor; an anesthesia cart; a bedside monitor; a non-invasive blood pressure monitor; and a non-invasive gas monitor.

11. A method (600) of regulating delivery of a fluid flow in a medical device coupled to a patient, the method comprising:
flowing (610) at least a first fluid input through a first pump of a fluid flow system along a fluid pathway to generate a first intermediate fluid flow;
flowing (620) at least a portion of the first intermediate fluid flow through a second pump of the fluid flow system along the fluid pathway to generate a second intermediate fluid flow;
flowing a second fluid input through a check valve system along the fluid pathway between the first pump and the second pump such that the second pump received the first intermediate fluid flow and the second fluid input; and
outputting (640) from the fluid flow system a fluid output to an inflatable device connected to the fluid flow system, the fluid output comprising the second intermediate fluid flow;
wherein the fluid output has an output flowrate such that the inflatable device reaches a target pressure within a target duration.

12. The method of claim 11, further comprising a step (630) including:
diverting at least a portion of the first intermediate fluid flow through a flow bypass along the flow pathway; and
introducing the diverted portion of the first intermediate fluid flow back into a downstream portion of the flow pathway located after the second pump;
wherein the fluid output comprises the second intermediate fluid flow and the diverted portion of the first intermediate fluid flow.

## Patentansprüche

1. Fluidströmungssystem (100, 100A, 100B) zur Verwendung in einer medizinischen Vorrichtung (500), wobei das Fluidströmungssystem Folgendes umfasst:
einen Strömungsweg, der einen oder mehrere Fluideingänge (104, 106) und einen Fluidausgang (108) umfasst, wobei der Fluidausgang mit einer aufblasbaren Vorrichtung (114) verbindbar ist, die so konfiguriert ist, dass sie den Fluidausgang aufnimmt;
eine erste Pumpe (202, 302), die so konfiguriert ist, dass sie einen ersten Fluideingang (104) aufnimmt und einen ersten Zwischenfluidstrom (206) ausgibt; und
eine zweite Pumpe (204, 304), die betriebsmäßig in Reihe mit der ersten Pumpe verbunden ist, wobei die zweite Pumpe so konfiguriert ist, dass sie zumindest einen Abschnitt des ersten Zwischenfluidstroms aufnimmt und einen zweiten Zwischenfluidstrom (201, 310) ausgibt;
wobei der Strömungsweg einen zweiten Fluideingang (106) zwischen der ersten Pumpe und der zweiten Pumpe beinhaltet, wobei die zweite Pumpe so konfiguriert ist, dass sie den ersten Zwischenfluidstrom und den zweiten Fluideingang aufnimmt; und wobei der Fluidausgang den zweiten Zwischenfluidstrom umfasst und eine Ausgangsströmungsgeschwindigkeit aufweist, sodass die aufblasbare Vorrichtung innerhalb einer Zieldauer einen Zieldruck erreicht,
**dadurch gekennzeichnet, dass** es weiter ein Rückschlagventilsystem (212) entlang des Strömungswegs zwischen der ersten Pumpe und der zweiten Pumpe umfasst, wobei das Rückschlagventilsystem so konfiguriert ist, dass es den zweiten Fluideingang reguliert und einen Rückfluss entlang des Strömungswegs verhindert.

2. Fluidströmungssystem nach Anspruch 1, wobei die erste Pumpe und die zweite Pumpe zur Verwendung in Magnetfeldern über 1000 Gauß ausgelegt sind.

3. Fluidströmungssystem nach Anspruch 1, wobei mindestens eine der ersten Pumpe und zweiten Pumpe eine piezoelektrische Pumpe ist.

4. Fluidströmungssystem nach Anspruch 1, wobei die erste Pumpe einen ersten Spitzendruck aufweist, der größer ist als ein zweiter Spitzendruck der zweiten Pumpe, die erste Pumpe eine erste maximale Durchflussrate aufweist, die kleiner ist als eine zweite maximale Durchflussrate der zweiten Pumpe, und der Zieldruck jeweils größer ist als der erste Spitzendruck und der zweite Spitzendruck.

5. Fluidströmungssystem nach Anspruch 1, wobei der Strömungsweg einen Strömungsumgehungskanal (314) zwischen der ersten Pumpe und der zweiten Pumpe beinhaltet, wobei der Strömungsumgehungskanal so konfiguriert ist, dass er zumindest einen Abschnitt des ersten Zwischenfluidstroms vor der zweiten Pumpe umleitet und den umgeleiteten Abschnitt des ersten Zwischenfluidstroms in einen stromabwärts gelegenen Abschnitt des Strömungswegs zurückführt, der sich nach der zweiten Pumpe befindet, so dass der Fluidausgang den zweiten Zwischenfluidstrom und den umgeleiteten Teil des ersten Zwischenfluidstroms umfasst.

6. Fluidströmungssystem nach Anspruch 1, das weiter ein Rückschlagventilsystem entlang des Strömungsumgehungskanals des Strömungswegs (314) umfasst, wobei das Rückschlagventilsystem so konfiguriert ist, dass es den umgeleiteten Abschnitt des ersten Zwischenfluidstroms reguliert und einen Rückfluss entlang des Strömungswegs verhindert.

7. Fluidströmungssystem nach Anspruch 6, wobei die erste Pumpe einen ersten Spitzendruck aufweist, der kleiner ist als ein zweiter Spitzendruck der zweiten Pumpe, die erste Pumpe eine erste maximale Durchflussrate aufweist, die größer ist als eine zweite maximale Durchflussrate der zweiten Pumpe, und der Zieldruck jeweils größer ist als der erste Spitzendruck und der zweite Spitzendruck.

8. Fluidströmungssystem nach Anspruch 1, wobei der Zieldruck mindestens etwa 250 mmHg beträgt und die Zieldauer höchstens etwa 30 Sekunden beträgt.

9. Medizinische Vorrichtung (500), die ein Fluidströmungssystem (100, 100A, 100B) nach den Ansprüchen 1-8, das so konfiguriert ist, dass es einen Fluidausgang erzeugt, und eine aufblasbare Vorrichtung (114) umfasst, die so konfiguriert ist, dass sie den Fluidausgang aufnimmt, wobei das Fluidströmungssystem betriebsmäßig mit der aufblasbaren Vorrichtung verbunden ist.

10. Medizinische Vorrichtung nach Anspruch 9, wobei die medizinische Vorrichtung mindestens eine der folgenden ist: ein MRI-kompatibler medizinischer Monitor; ein Anästhesiewagen; ein Monitor am Krankenbett; ein nichtinvasiver Blutdruckmonitor; und ein nichtinvasiver Gasmonitor.

11. Verfahren (600) zum Regulieren einer Abgabe eines Fluidstroms in einer medizinischen Vorrichtung, die an einen Patienten angeschlossen ist, wobei das Verfahren Folgendes umfasst:
Strömen (610) mindestens eines ersten Fluideingangs durch eine erste Pumpe eines Fluidströmungssystems entlang eines Fluidwegs, um einen ersten Zwischenfluidstrom zu erzeugen;
Strömen (620) mindestens eines Abschnitts des ersten Zwischenfluidstroms durch eine zweite Pumpe des Fluidstromsystems entlang des Fluidwegs, um einen zweiten Zwischenfluidstrom zu erzeugen;
Strömen eines zweiten Fluideingangs durch ein Rückschlagventilsystem entlang des Fluidwegs zwischen der ersten Pumpe und der zweiten Pumpe, so dass die zweite Pumpe den ersten Zwischenfluidstrom und den zweiten Fluideingang aufnimmt; und
Ausgeben (640) eines Fluidausgangs aus dem Fluidströmungssystem an eine aufblasbare Vorrichtung, die mit dem Fluidströmungssystem verbunden ist, wobei der Fluidausgang den zweiten Zwischenfluidstrom umfasst;
wobei der Fluidausgang eine solche Ausgangsströmungsgeschwindigkeit aufweist, dass die aufblasbare Vorrichtung innerhalb einer Zieldauer einen Zieldruck erreicht.

12. Verfahren nach Anspruch 11, weiter umfassend einen Schritt (630), der Folgendes beinhaltet:
Umleiten zumindest eines Abschnitts des ersten Zwischenfluidstroms durch einen Strömungsumgehungskanal entlang des Strömungswegs; und
Einleiten des umgeleiteten Abschnitts des ersten Zwischenfluidstroms zurück in einen stromabwärts gelegenen Abschnitt des Strömungswegs, der sich hinter der zweiten Pumpe befindet;
wobei der Fluidausgang den zweiten Zwischenfluidstrom und den umgeleiteten Abschnitt des ersten Zwischenfluidstroms umfasst.

## Revendications

1. Système d'écoulement de fluide (100, 100A, 100B) destiné à être utilisé dans un dispositif médical (500), le système d'écoulement de fluide comprenant :
un trajet d'écoulement comprenant une ou plusieurs entrées de fluide (104, 106) et une sortie de fluide (108), la sortie de fluide pouvant être raccordée à un dispositif gonflable (114) configuré pour recevoir la sortie de fluide ;
une première pompe (202, 302) configurée pour recevoir une première entrée de fluide (104) et délivrer un premier écoulement de fluide intermédiaire (206) ; et
une seconde pompe (204, 304) raccordée de manière opérationnelle en série à la première pompe, la seconde pompe étant configurée pour recevoir au moins une partie du premier écoulement de fluide intermédiaire et délivrer un second écoulement de fluide intermédiaire (201, 310) ;
dans lequel le trajet d'écoulement inclut une seconde entrée de fluide (106) entre la première pompe et la seconde pompe, dans lequel la seconde pompe est configurée pour recevoir le premier écoulement de fluide intermédiaire et la seconde entrée de fluide ; et dans lequel la sortie de fluide comprend le second écoulement de fluide intermédiaire et présente un débit de sortie tel que le dispositif gonflable atteigne une pression cible dans une durée cible,
**caractérisé en ce qu'**il comprend en outre un système de clapet anti-retour (212) le long du trajet d'écoulement entre la première pompe et la seconde pompe, le système de clapet anti-retour étant configuré pour réguler la seconde entrée de fluide et empêcher le reflux le long du trajet d'écoulement.

2. Système d'écoulement de fluide selon la revendication 1, dans lequel la première pompe et la seconde pompe sont conçues pour être utilisées dans des champs magnétiques dépassant 1000 gauss.

3. Système d'écoulement de fluide selon la revendication 1, dans lequel au moins l'une de la première pompe et de la seconde pompe est une pompe piézoélectrique.

4. Système d'écoulement de fluide selon la revendication 1, dans lequel la première pompe présente une première pression de pointe qui est supérieure à une seconde pression de pointe de la seconde pompe, la première pompe présente un premier débit maximal qui est inférieur à un second débit maximal de la seconde pompe, et la pression cible est supérieure à chacune de la première pression de pointe et de la seconde pression de pointe.

5. Système d'écoulement de fluide selon la revendication 1, dans lequel le trajet d'écoulement inclut une dérivation d'écoulement (314) entre la première pompe et la seconde pompe, la dérivation d'écoulement étant configurée pour dévier au moins une partie du premier écoulement de fluide intermédiaire avant la seconde pompe et renvoyer la partie déviée du premier écoulement de fluide intermédiaire dans une partie en aval du trajet d'écoulement située après la seconde pompe de telle sorte que la sortie de fluide comprenne le second écoulement de fluide intermédiaire et la partie déviée du premier écoulement de fluide intermédiaire.

6. Système d'écoulement de fluide selon la revendication 1, comprenant en outre un système de clapet anti-retour le long de la dérivation d'écoulement du trajet d'écoulement (314), le système de clapet anti-retour étant configuré pour réguler la partie déviée du premier écoulement de fluide intermédiaire et empêcher le reflux le long du trajet d'écoulement.

7. Système d'écoulement de fluide selon la revendication 6, dans lequel la première pompe présente une première pression de pointe qui est inférieure à une seconde pression de pointe de la seconde pompe, la première pompe présente un premier débit maximal qui est supérieur à un second débit maximal de la seconde pompe, et la pression cible est supérieure à chacune de la première pression de pointe et de la seconde pression de pointe.

8. Système d'écoulement de fluide selon la revendication 1, dans lequel la pression cible est d'au moins environ 250 mmHg et la durée cible est d'au plus environ 30 secondes.

9. Dispositif médical (500) comprenant un système d'écoulement de fluide (100, 100A, 100B) selon les revendications 1-8, configuré pour produire une sortie de fluide et un dispositif gonflable (114) configuré pour recevoir la sortie de fluide, le système d'écoulement de fluide étant raccordé de manière opérationnelle au dispositif gonflable.

10. Dispositif médical selon la revendication 9, dans lequel le dispositif médical est au moins l'un parmi : un moniteur médical compatible IRM ; un chariot d'anesthésie ; un moniteur de chevet ; un moniteur de pression artérielle non invasif ; et un moniteur de gaz non invasif.

11. Procédé (600) de régulation de l'administration d'un écoulement de fluide dans un dispositif médical couplé à un patient, le procédé comprenant :
l'écoulement (610) d'au moins une première entrée de fluide à travers une première pompe d'un système d'écoulement de fluide le long d'un trajet de fluide pour générer un premier écoulement de fluide intermédiaire ;
l'écoulement (620) d'au moins une partie du premier écoulement de fluide intermédiaire à travers une seconde pompe du système d'écoulement de fluide le long du trajet de fluide pour générer un second écoulement de fluide intermédiaire ;
l'écoulement d'une seconde entrée de fluide à travers un système de clapet anti-retour le long du trajet de fluide entre la première pompe et la seconde pompe de telle sorte que la seconde pompe reçoive le premier écoulement de fluide intermédiaire et la seconde entrée de fluide ; et
la délivrance (640), à partir du système d'écoulement de fluide, d'une sortie de fluide vers un dispositif gonflable raccordé au système d'écoulement de fluide, la sortie de fluide comprenant le second écoulement de fluide intermédiaire ;
dans lequel la sortie de fluide présente un débit de sortie tel que le dispositif gonflable atteigne une pression cible dans une durée cible.

12. Procédé selon la revendication 11, comprenant en outre une étape (630) incluant :
la déviation d'au moins une partie du premier écoulement de fluide intermédiaire à travers une dérivation d'écoulement le long du trajet d'écoulement ; et
l'introduction de la partie déviée du premier écoulement de fluide intermédiaire dans une partie en aval du trajet d'écoulement située après la seconde pompe ;
dans lequel la sortie de fluide comprend le second écoulement de fluide intermédiaire et la partie déviée du premier écoulement de fluide intermédiaire.
